# EUROPÄISCHE PATENTSCHRIFT

(11) **EP 0 411 265 B1**
(45) Veröffentlichungstag und Bekanntmachung des Hinweises auf die Patenterteilung: **18.08.1993**
(21) Anmeldenummer: 90110165.9
(22) Anmeldetag: 29.05.1990
(51) Int. Cl.: A61M 25/01

(54) **Drainage-Set für Ureter-Drainage**
Drainage set for ureter drainage
Ensemble de drainage pour l'urètre

(30) Priorität: 29.07.1989 DE 3925263
(43) Veröffentlichungstag der Anmeldung: 06.02.1991
(73) Patentinhaber: ANGIOMED Aktiengesellschaft, 76229 Karlsruhe (DE)
(72) Erfinder: Schnepp-Pesch, Wolfram, D-7505 Ettlingen (DE); Lindenberg, Josef, D-7500 Karlsruhe 1 (DE)
(74) Vertreter: Dipl.-Ing. Heiner Lichti Dipl.-Phys. Dr.rer.nat. Jost Lempert Dipl.-Ing. Hartmut Lasch

(56) Entgegenhaltungen:
- EP-A- 0 313 807
- FR-A- 2 557 460
- US-A- 2 024 982

## Beschreibung

Die Erfindung betrifft ein Drainage-Set, insbesondere zur Ureter-Drainage, mit einer Drainage-Schiene, einem Führungsmandrin und einem Schieber.

Im Rahmen der Erfindung werden als Drainage-Schienen solche wie Ureter-Schienen, Katheter oder dergleichen verstanden, die aus relativ flexiblem Material, wie Kunststoff oder Silikon bestehen. Die Drainage-Schienen haben die Form eines zumindest einen gestreckten Abschnitt aufweisenden, flexiblen langen Hohlkörpers mit in der Mantelwandung angeordneten Drainageöffnungen und sind zumindestens proximal stirnseitig offen. Führungsdrähte bestehen vorzugsweise aus Metall, wie Edelstahl, und sind als Wendel mit einem axialen Hohlraum ausgebildet. Schieber sind Rohre, vorzugsweise Kunststoffrohre und besitzen insbesondere eine hinreichende axiale Steifigkeit. Es ist bekannt, im Körper verbleibende Ureter-Schienen in den Harnleiter einzusetzen, um bei Verengungen, Fisteln oder dergleichen den Abfluß aus der Niere sicherzustellen. Zur Festlegung der Schiene wurden Dislokationssicherungen in Form von Widerhaken am Außenumfang des Schienenkörpers oder aber Krümmungen oder hakenartige Ausbildungen der Schiene selbst an einem oder beiden ihrer Enden vorgesehen (Pig-Tail-Katheter). Die Schienen konnten derart eingeführt werden, daß entweder zunächst ein Führungsmandrin in den Ureter eingeführt wird und anschließend die Schiene über diesen in den Katheter eingeschoben wird oder aber alternativ hierzu die Schiene nur mit einem offenen Ende (dem proximalen Ende) versehen ist, während das distale Ende geschlossen ist. In diesem Fall wurde die Schiene über den Führungsmandrin geschoben, der aufgrund seiner relativen Steifheit das oder die Hakenenden streckt, so daß die Schiene dann mit eingeführtem Führungsmandrin und unter Vorschieben desselben gelegt werden konnte. Nachteilig ist in beiden Fällen, daß eine angulare Ausrichtung der Schiene nicht möglich ist. Führungsmandrin und Schiene sind relativ zueinander verdrehbar. Auch kann in gestrecktem Zustand ohne besondere Markierungen an der Schiene, wie röntgen- oder ultraschallsichtbare Markierungen, die Ausrichtung der Schiene vor Herausnehmen des Führungsmandrins nicht, mit Markierungen nur schlecht, festgestellt werden. Ein weiterer Nachteil der unter Vorschieben des Führungsmandrins eingeführten Schiene besteht darin, daß aufgrund der geschlossenen distalen Stirnseite die Drainage nicht so optimal ist, wie sie sein könnte und bei offener Stirnseite ist.

Aus der FR-A-25 57 460 ist ein Drainage-Set mit einer vorstehend beschriebenen Schiene mit einer kleinen vorderen Öffnung sowie einem Schieber bekannt. Das Set weist weiterhin einen Führungsmandrin auf, dessen Querschnitt geringer ist als der der vorderen Öffnung der Schiene, so daß über diesen Führungsmandrin die Schiene in Seldinger-Technik eingeführt werden kann. Das Set weist zusätzlich einen Führungsmandrin mit einem größeren Durchmesser als die vordere Öffnung der Schiene auf, so daß die Schiene unter Vorschieben des Führungsmandrins eingeschoben werden kann. Auch für dieses Set gilt insbesondere, daß eine angulare Ausrichtung der Schiene nicht möglich ist. Darüber hinaus ist ein Zurückziehen der Schiene,falls diese zu weit vorgeschoben wurde, nicht möglich und damit ein genaues longitudinales Anordnen nicht in jedem Fall gewährleistet.

Aus der DE-OS 35 11 448 ist ein hohler Führungsmandrin für Drainageschienen bekanntgeworden, der ein offenes distales Ende aufweist und in seinem Mantel achsparallel ein- oder mehrfach gespalten ist. Durch den Mandrin ragt ein Draht, der fest mit einem aus dem distalen Ende des Mandrin herausragenden Keil verbunden ist. Der Keil ist durch den Draht in den Mandrin hereinziehbar, so daß er das Ende des Mandrins aufweitet, wodurch dieses zur Anlage an der Innenwand der Drainageschiene gelangen kann. Hierdurch ist zwar theoretisch auch ein Zurückziehen und Verdrehen der Drainageschiene im Ureter möglich, es bestehen aber Bedenken hinsichtlich der praktischen Verwirklichbarkeit, da der Keil, wenn er in den Ureter hereingezogen ist, mit erheblicher Kraft kraftschlüssig in diesem festsitzt und Bedenken bestehen, daß er durch den mit ihm verbundenen dünnen Draht entgegen diesem Kraftschluß aus dem Führungsmandrin ausgeschoben werden kann, wenn man bedenkt, daß ein solcher Mandrin einen Innendurchmesser im Bereich von wenigen Zehntelmillimetern aufweist, aber eine Länge von bis zu 1 m; der mit dem Keil verbundene Draht dürfte nicht mit einer ausreichenden axialen Steifigkeit erhaltbar sein.

Darüberhinaus kann diese Ausgestaltung die Abbiegung des distalen Endes der Drainageschiene nicht zwischen verschiedenen Winkeln kontinuierlich steuern. Auch ist das Steuern und Legen von distal offenen Schienen zur optimalen Drainage nicht möglich, da das offene distale Ende des Führungsmandrins und die Kanten der Schlitze Verletzungsgefahren bedingen.

Der Erfindung liegt daher die Aufgabe zugrunde, ein Drainage-Set zu schaffen, das bei einfachster Ausgestaltung eine optimale Steuerung der Schiene hinsichtlich ihrer angularen Ausrichtung, aber auch ein einfaches Zurückziehen und gegebenenfalls Entfernen der Schiene - unter Freigabe derselben - und insbesondere das Legen von distal offenen Schienen mit optimalen Drainagen ermöglicht.

Erfindungsgemäß wird die genannte Aufgabe durch ein Drainage-Set gelöst, welches dadurch gekennzeichnet ist, daß der Führungsmandrin eine fest auf ihm ausgebildete Verdickung aufweist, auf der die Drainage-Schiene mit ihrem Lumen reibschlüssig aufschiebbar ist, und daß der Lumendurchmesser des Schiebers größer oder gleich dem Durchmesser der Verdickung, aber geringer als der Außendurchmesser der Drainage-Schiene ist.

Die Erfindung schafft die Möglichkeit zur radialen kraftschlüssigen Verspannung des in die Schiene eingeführten Führungsmandrins bzw. eines Führungsmandrinabschnitts und der Schiene.

Das erfindungsgemäße Drainage-Set wird derart eingesetzt, daß zunächst die Schiene mit ihrem proximalen Ende vom distalen Ende des Führungsmandrines über diesen geschoben wird, bis ihr proximales Ende zur Verdickung gelangt. Das proximale Ende der Schiene wird dann kraftschlüssig über die Verdickung des Führungsmandrines geschoben und derart kraftschlüssig axial und angular relativ fest mit dem Führungsmandrin verbunden. Hierdurch werden aufgrund der Steifigkeit des Führungsmandrines die Enden der Drainage-Schiene gestreckt, so daß diese mittels des Führungsmandrines durch ein Zystoskop in den Ureter und durch diesen geschoben werden kann. Jetzt oder auch vorher wird vom proximalen Ende des Führungsmandrines her über diesen ein Schieber geschoben, der unter Gegenhaltung mit seinem distalen Ende am proximalen Ende der Schiene angreift und diese von der Verdickung des Führungsmandrines herunterschieben kann. Dabei ist es wichtig, daß der Innendurchmesser des Schiebers und der querschnitt der Verdickung angepaßt sind, so daß die Verdickung axial im Schieber verschiebbar ist. Der Führungsmandrin kann anschließend herausgezogen werden, wodurch die Biegekräfte der Enden der Drainage-Schiene freigegeben werden, so daß sich die Ursprungshaken derselben wieder bilden können, die Dislokationssicherungen der Drainage-Schiene, wie bei einer Ureter-Schiene in der Niere und in der Uretra, bilden. Aufgrund der angularen und axialen kraftschlüssigen Verbindung zwischen Verdickung des Führungsmandrines und Drainage-Schiene kann letztere nicht nur eingeschoben, sondern auch zurückgeschoben und mittels des Führungsmandrines angular verdreht werden. Letzteres ist insbesondere vorteilhaft, wenn der Führungsmandrin hohl ausgebildet ist und sich durch ihn eine axial relativ verschiebbare Seele erstreckt, wobei insbesondere die Seele proximal mit einem Handhabungsansatz fest verbunden ist, der eine der Schiene entsprechende Stärke aufweist und die Seitenelastizität des Führungsmandrins in seinem distalen Endbereich bei aus diesem Bereich herausgezogener Seele geringer ist als die Rückstellkraft des hakenförmigen distalen Endes der Drainage-Schiene. Hierdurch kann durch Zurückziehen der Seele im Führungsmandrin die Steifigkeit des distalen Endes desselben reduziert werden, so daß aufgrund der seitlichen Eigenbiegekräfte der Drainage-Schiene deren distales Ende und das distale Ende des Führungsmandrines leicht zur Seite geneigt werden, so daß das Drainage-Set mit der Schiene durch Verdrehen des Führungsmandrines um ein Hindernis herumgesteuert werden kann.

Bei einer Ausgestaltung in der Art, daß die Verdickung eine n-zählige Rotationssymmetrie parallel zur Längsachse des Führungsmandrins aufweist, wobei n eine natürliche endliche Zahl ist, oder daß die Verdickung einen mehrkantigen Querschnitt aufweist, wird ein besonders drehfester Reibschluß zwischen der Verdickung und der Drainage-Schiene gewährleistet. Somit wird der angulare Reibschluß zwischen der Verdickung und der Drainage-Schiene erheblich gesteigert. Zudem hat es sich in Verbindung mit den beiden vorgenannten Ausführungen als sehr vorteilhaft erwiesen, wenn der Schieber so ausgebildet ist, daß die Kontur des lichten Querschnitts des Schiebers an seinem distalen Ende der Kontur der Verdickung angepaßt ist, so daß eine in das distale Ende des Schiebers eingesteckte Verdickung zwar axial verschieblich aber drehfest mit diesem verbunden ist. Dadurch ist es möglich, die Verdickung mit dem Schieber zu verdrehen, wenn die Verdickung zumindest teilweise in das distale Ende des Schiebers eingesteckt ist.

Mit dieser Ausbildung ist es also möglich, eine drehfeste Verbindung des Schiebers mit der Drainage-Schiene herzustellen. Daraus ergibt sich der Vorteil, daß der Führungsmandrin nicht so drehfest und dementsprechend dünner ausgebildet sein kann.

Weitere bevorzugte Ausgestaltungen sehen vor, daß die Verdickung aus einer am Führungsmandrin befestigten, diesen umgebenden Hülse besteht, wobei entweder vorgesehen sein kann, daß die Verdickung einstückig mit dem Führungsmandrin ausgebildet ist oder daß die Verdickung durch Kleben mit dem Führungsmandrin verbunden ist oder aber daß die Verdickung durch Anschmelzen und anschließendem Erstarren mit dem Führungsmandrin verbunden ist.

Andere bevorzugte Merkmale sind darin zu sehen, daß die Verdickung an ihren Stirnseiten sich konisch verjüngt, daß die Drainage-Schiene im unbelasteten Zustand zumindestens ein, vorzugsweise zwei gekrümmte Ende(n) aufweist und/oder insbesondere daß die Drainage-Schiene ein stirnseitig offenes distales Ende aufweist.

Weitere Vorteile und Merkmale der Erfindung ergeben sich aus den Ansprüchen und aus der nachfolgenden Beschreibung, in der bevorzugte Ausführungsformen der Erfindung unter Bezugnahme auf die Zeichnung im einzelnen erläutert sind. Dabei zeigt:
- Figur 1: Eine Drainage-Schiene als Teil des erfindungsgemäßen Drainage-Sets;
- Figur 2: ein erfindungsgemäß ausgebildeter Führungsmandrin als Teil des Drainage-Sets;
- Figur 3: einen Schieber des erfindungsgemäßen Drainage-Sets;
- Figur 4: eine vergrößerte Ausschnittsdarstellung des erfindungsgemäßen Drainage-Sets bei auf die Verdickung aufgeschobener Drainage-Schiene mit angesetztem Schieber;
- Figur 5: Führungsmandrin mit aufgeschobener Ureter-Schiene bei eingeschobener Seele;
- Figur 6: den Gegenstand der Figur 5 bei zurückgezogener Seele;
- Figur 7: eine Ausschnittdarstellung des proximalen Endes einer proximal offenen Ureter-Schiene mit aus dessen offenem Ende herausragenden proximalen Ende des Führungsmandrins;
- Figur 8: wie Figur 4 jedoch verbindet die Verdickung die Drainage-Schiene und den Schieber;
- Figur 9: Schnitt von Figur 8 und
- Figur 10: ein weiterer Querschnitt der Verdickung.

Das erfindungsgemäße Drainage-Set weist eine Drainage-Schiene 3, einen Führungsdraht als Führungsmandrin 4 - in Form eines eng gewickelten Spiraldrahtes - und einen Schieber 5 auf.

Die Drainage-Schiene, wie Ureter-Schiene kann zystoskopisch, perkutan oder intraoperativ eingesetzt werden. Eine solche Ureter-Schiene ist an sich bekannt, wobei sie aus einem langgestreckten Hauptteil 31 besteht, das an seinem distalen Ende 34 und seinem proximalen Ende 36 mit einem Bogen oder Haken 32,33 versehen ist. Im Bereich der beiden Haken 32,33 sind in der seitlichen Wandung zum inneren Hohlraum führende Drainage-Öffnungen 37 ausgebildet. Ebenfalls könnten am langgestreckten Hauptteil 31 Drainage-Öffnungen ausgebildet sein. Weiterhin kann eine solche Schiene umlaufende Rillen oder Erhebungen 35 aufweisen, die über die Länge hin in verschiedener Anzahl gruppiert sind. Während bekannte Ureter-Schienen zumindestens an einem Ende, nämlich dem distalen Ende, vollständig geschlossen sind und sein müssen, damit sie durch einen eingeführten Führungsmandrin eingeschoben werden können und während eine solche, an sich bekannte Ureterschiene ebenfalls bei der Erfindung verwendet werden kann und Teil des erfindungsgemäßen Drainage-Sets sein kann, sieht eine äußerst bevorzugte Ausgestaltung vor, daß die Schiene an beiden Stirnseiten offen ist. Hierdurch wird der herstellungsmäßige Vorteil erreicht, daß die Schiene 3 vom Endlosschlauchmaterial abgeschnitten werden kann, ohne daß ein Ende in einem separaten Schnitt noch verschlossen werden muß. Darüberhinaus liegt ein weiterer wesentlicher Vorteil in der besseren Drainage-Wirkung durch eine distale stirnseitige Öffnung des Hohlraums der Schiene 3.

Der Führungsmandrin 4 weist eine Verdickung 41 auf. Die Verdickung 41 ist vorzugsweise etwa 1 cm lang und weist einen Durchmesser auf, der etwas über dem Durchmesser des Lumens der Ureter-Schiene 3 liegt, so daß diese auf die Verdickung 41 mit ihrem proximalen Ende 36 kraftschlüssig aufschiebbar ist. Der Durchmesser der Verdickung kann dabei von 1 bis 2 mm je nach Stärke der Schiene 3 liegen. Die Verdickung 41 befindet sich vom distalen Ende 42 des Mandrins 4 in einem Abstand, der im wesentlichen der Länge der Schiene 3 entspricht und bei geschlossener Schiene etwas unterhalb der Länge derselben, bei offener Schiene etwas oberhalb, beispielsweise 1 cm oberhalb der Länge der Schiene 3, liegt.

Die Verdickung 41 ist fest mit dem Mandrin 4 verbunden. Die Verdickung 41 kann einstückig mit dem Mandrin 4 ausgebildet sein. Im dargestellten Ausführungsbeispiel besteht sie aus einer über den Mandrin 4 geschobenen Hülse, die mit diesem fest verklebt ist. Stattdessen könnte eine Kunststoffhülse auch nach Aufschieben bis auf den gewünschten Ort auf dem Führungsmandrin 4 durch Erwärmen und Aufschmelzen mit diesem ohne Klebstoff fest verbunden sein. Die Verbindung kann in diesem Falle vorzugsweise dadurch vorgenommen werden, daß der Mandrin 4 und damit die die Verdickung 41 bildende Hülse von innen her erhitzt wird, beispielsweise durch resistive oder induktive Erhitzung des als Draht ausgebildeten Mandrins 4.

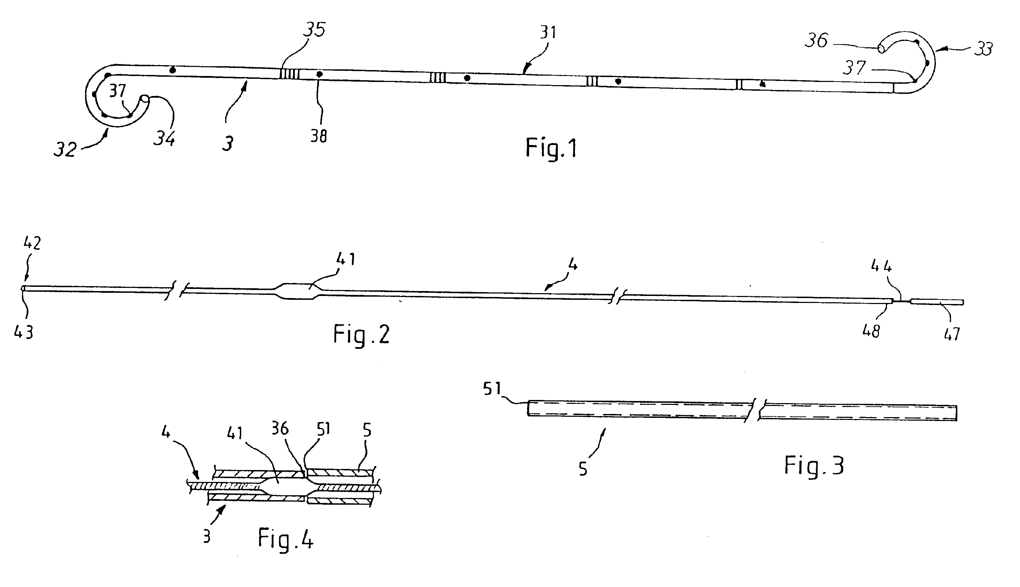

Der Mandrin 4 ist vorzugsweise hohl ausgebildet, beispielsweise als in an sich bekannter Weise gewickelte Drahtwendel, die axial durch einen zumindestens an ihren beiden Enden festgelegten Flachdraht zusammengehalten wird. Das distale Ende 42 ist abgerundet, beispielsweise mit einer Kuppe 43 versehen. Durch den durch die Wendel des Führungsmandrins 4 gebildeten Hohlraum erstreckt sich frei bzw. reibschlüssig eine Seele 44, die am proximalen Ende 46 des Mandrins 4 mit einem Handhabungsansatz 47 fest verbunden ist, der die gleiche Stärke wie der eigentliche Führungsmandrin 4 aufweist. Mittels des Ansatzes 47 kann die Seele 44 im Führungsmandrin 4 relativ zu diesem axial verschoben werden. Der Sinn dieser Ausgestaltung wird weiter unten erläutert. Weiter gehört zum erfindungsgemäßen Drainage-Set ein Schieber 5 (Figur 3). Es handelt sich hierbei um ein Kunststoffhohlrohr, das an beiden Enden offen ist. Der Innendurchmesser des Schiebers 5 ist größer als der Durchmesser der Verdickung 41, so daß der Schieber 5 frei ohne Reibung über die Verdickung 41 gleiten kann. Der Innendurchmesser des Schiebers 5 ist aber geringer als der Außendurchmesser der Schiene 3, so daß der Schieber 5 stirnseitig an der Schiene 3 angreifen und die Schiene 3 mittels des Schiebers verschoben werden kann.

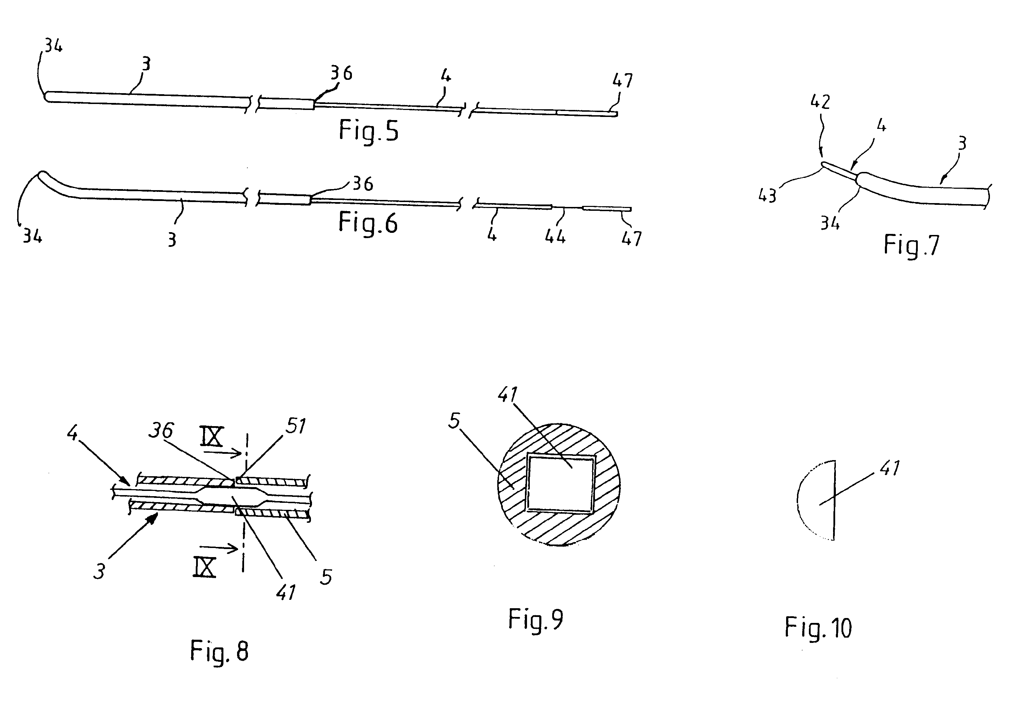

Während in den Figuren 1 bis 3 die einzelnen Teile des erfindungsgemäßen Drainage-Sets separat dargestellt sind, zeigt die Figur 4 eine Ausschnittsdarstellung des zum Einsatz zusammengesetzten Drainage-Sets. Hierbei ist die Schiene 3 vom distalen Ende 43 des Führungsmandrins 4 her über diesen bis zur Verdickung 41 und über diese geschoben. Die Verdickung 41 ist an ihren Stirnseiten konisch ausgebildet. Da der Durchmesser der Verdickung 41 leicht über dem Innendurchmesser des Lumens der Schiene 3 liegt, sitzt diese kraftschlüssig auf der Verdickung 41 auf. Die beiden Hakenausbildungen 32,33 der Schiene 3 sind dabei durch die Eigensteifigkeit des Führungsmandrins 4 (gegebenenfalls aufgrund voll in diesen eingeschobener Seele 44) gestreckt oder aufgestellt, so daß die Schiene 3 eine gerade gestreckte Form aufweist, wie sie in der Figur 5 dargestellt ist. In diesem Zustand kann die Schiene durch ein Zystoskop in den zu drainierenden Hohlraum, wie den Ureter, eingeschoben werden. Um die Schiene 3 von der Verdickung 41 und damit vom Mandrin 4 zu lösen, so daß dieser nach Legen der Schiene zurückgezogen werden kann, wird der Schieber 5 über den Mandrin 4 (von dessen proximalem Ende 48 her) verschoben, bis seine distale Stirnseite 51 zur Anlage an der proximalen Stirnseite 36 der Schiene 3 gelangt. Bei weiterem Vorschieben des Schiebers 5 unter Gegenhaltung des Führungsmandrins 4 am proximalen Ende 48 (Figur 2) kann die Schiene 3 von der Verdickung 41 durch die Stirnseite 51 des Schiebers 5 abgeschoben werden oder der Schieber 5 wird gegengehalten und der Führungsmandrin 4 mit seiner Verdickung 41 wird zurückgezogen, wobei der Schieber 5 ein Zurückziehen der Drainage-Schiene 3 verhindert. Hierdurch wird der Kraft- oder Reibschluß zwischen Schiene 3 und der Verdickung 41 des Führungsmandrins 4 gelöst. Anschließend kann der Führungsmandrin 4 frei, gegebenenfalls durch den Schieber 5, aus dem Körperhohlraum herausgezogen werden.

Beim Einschieben des Führungsmandrins 4 in die Schiene 3 von deren proximalen Ende 36 her wird der Führungsmandrin bis in den Bereich des distalen Hakens oder der Krümmung 32 am distalen Ende 34 eingeschoben, wobei der Führungsmandrin 4 aufgrund seiner Eigensteifigkeit, wie gesagt, den Haken 32 in an sich bekannter Weise aufstellt, so daß dann das distale Ende 34 der Schiene die zum Einführen erforderliche wesentlich gestreckte Ausgestaltung aufweist (Figur 5).

Die Verdickung kommt dadurch zur Anlage an der Innenwandung der Schiene 3 im Bereich des gestreckten Teils 31, beult diese leicht aus, schafft insgesamt aber eine gut reib- bzw. kraftschlüssige Verbindung mit der Innenwandung der Schiene 3. Hiernach kann die Schiene 3 dann gelegt werden, indem sie mittels des Führungsmandrins 4 beispielsweise durch die Harnröhre, die Blase, in den Harnleiter und durch diesen hindurch mit ihrem distalen Ende 34 bis zur Niere geführt wird. Dies kann unter Beobachtung, wie unter Ultraschall- oder Röntgen-Beobachtung, erfolgen. Die Position der Schiene 3 kann wiederholt korrigiert werden. Insbesondere kann sie aufgrund der reibschlüssigen Verbindung zwischen der Verdickung 41 und damit der Einführvorrichtung und der Innenwandung der Schiene 3 nicht nur vorgeschoben, sondern auch zurückgezogen werden, um die Lage zu verbessern. Auch kann durch die reibschlüssige Verbindung die Schiene 3 verdreht werden, was beim Stand der Technik ebenfalls nicht möglich war. Der Reibschluß bezüglich des relativen Verdrehens der Verdickung 41 und der Schiene 3 wird dadurch erhöht, daß die Verdickung 41 mehrkantig ausgebildet ist. Eine bevorzugte Ausführung sieht vor, daß die Verdickung einen quadratischen Querschnitt aufweist (vergleiche Figur 9).

Wenn die Schiene 3 ihre richtige Position erreicht hat, so wird die Schiene 3 in der beschriebenen Weise mittels des Schiebers 5 von der Verdickung 41 des Führungsmandrins 4 ab gestreift. Anschließend kann der Führungsmandrin 4 aus der Schiene 3 frei zurückgezogen werden.

Wenn die Schiene 3 beim Einschieben in den Körperhohlraum, wie den Ureter, mit ihrem distalen Ende 34 an ein Hindernis, wie eine Stenose oder dergleichen anstößt, um das sie herumgeführt werden muß, so kann, wie oben erläutert, die den Führungsmandrin 4 aussteifende Seele 44 über den Griffansatz 47 in proximale Richtung relativ zum Mandarin 4 zurückgezogen werden. Hierdurch wird das distale Ende 42 des Mandrins 4 weicher und setzt damit der die Hakenform 32,34 anstrebenden Eigenelastizität der Schiene 3 weniger Widerstand entgegen, so daß das distale Ende 34 der Schiene 3 in der aus der Figur 6 ersichtlichen Form leicht zur Seite gebogen werden kann. Weiterhin kann aufgrund der reibschlüssigen Verbindung zwischen Schiene 3 und Führungsmandrin 4 (über die Verdickung 41) die Schiene 3 durch Verdrehen des Führungsmandrins 4 ebenfalls mit verdreht werden, so daß das leicht abgebogene distale Ende 34 (Figur 6) hierdurch angular ausgerichtet werden kann. Es kann daher eine solche angulare Ausrichtung vorgenommen werden, daß das abgebogene distale Ende 34 an dem Hindernis, wie einer Stenose, vorbeizeigt, so daß beim weiteren Einschieben das distale Ende 34 die Schiene 3 um das Hindernis bzw. an diesem vorbeiführen kann. Dies ist ein wesentlicher Vorteil gegenüber dem bekannten Drainage-Set, das mittels Pusher-Technik eingeführt wird, bei dem der Führungsmandrin mit seinem distalen Ende an der distalen Innenwandung der Schiene anliegt (damit er sie derart schieben kann) und Schiene und Führungsmandrin relativ zueinander verdrehbar sind, während bei dem erfindungsgemäßen Drainage-Set Schiene und Führungsmandrin durch die kraftschlüssige Verbindung über die Verdickung 41 nicht nur axial, sondern auch angular zueinander festgelegt sind.

Es kann vorkommen, daß Hindernisse, wie Stenosen, beispielsweise im Harnleiter, diesen so verschließen, daß das relativ breite distale Ende 34 der Schiene 3 sich durch den Verschluß keinen Weg schaffen kann. In diesem Falle ist es sinnvoll, eine Führungsschiene 3 mit einem stirnseitig offenen distalen Ende 34 zu verwenden und den Führungsmandrin 4 in und durch die Schiene 3 so weit hindurchzuschieben, daß das distale Ende 42 des Führungsmandrins 4 ein Stück aus dem distalen Ende 34 der Schiene 3 hinausragt, wie dies in Figur 7 dargestellt ist. Hierdurch wird eine gegenüber dem distalen Ende 34 der Schiene 3 wesentlich verjüngte Spitze des gesamten Drainage-Sets geschaffen (die durch die Kuppe 43 abgerundet ist). Hierdurch kann bei Verengung, beispielsweise Stenosen oder dergleichen, leichter ein Weg an dieser vorbei gefunden werden, da sich das gegenüber dem distalen Ende 34 der Schiene 3 dünnere distale Ende 42 des Führungsmandrins 4 leichter einen Weg durch die Verengung bahnen kann. Verletzungen werden durch die abgerundete Kuppe 43 des Führungsmandrins 4 vermieden. Eine solche distal offene Schiene 3 zeigt aufgrund des offenen distalen Endes 34 im übrigen eine verbesserte Drainagewirkung.

Das Herumsteuern der Schiene 3 um ein Hindernis wird im übrigen dadurch vereinfacht, daß bei der Abbiegung des distalen Endes 34 der Schiene 3 dieses unter Ultraschall deutlich erkennbar ist, während bei gestreckter Ausbildung der Haken 32,33 bei Einführen in herkömmlicher Weise diese nicht ohne weiteres unter Ultraschall erkennbar sind.

In einer weiteren bevorzugten Ausführung ist vorgesehen, daß die Verdickung 41 und das distale Ende 51 des Schiebers 5 so ausgebildet sind, daß diese im eingesteckten Zustand drehfest zusammenwirken. Es wird dadurch, wie in Figur 8 dargestellt, eine drehfeste Verbindung des Schiebers 5 mit der Schiene 3 über die Verdickung 41, die als Kuppelglied wirkt, ermöglicht. Dazu ist es notwendig, daß die Verdickung 41 zum einen den entsprechenden Querschnitt, um mit dem distalen Ende 51 des Schiebers 5 zusammenwirken zu können, und zum anderen die entsprechende Länge aufweist, um gleichzeitig einen ausreichenden Reibschluß mit der Schiene 3 und die drehfeste Verbindung mit dem Schieber 5 zu gewährleisten.

Um die Möglichkeit auszunutzen, die Schiene 3 mit dem im Gegensatz zum Führungsmandrin 4 wesentlich torsionsstabileren Schieber 5 angular zu verdrehen, wird die Schiene 3 mit ihrem proximalen Ende 36 auf den Führungsmandrin 4 vom distalen Ende 42 her bis etwa zur Hälfte der Ver dickung 41 aufgeschoben, wie in Figur 8 zu sehen ist. Das distale Ende 51 des Schiebers 5 wird auf den Führungsmandrin 4 vom proximalen Ende 48 her bis zur Hälfte der Verdickung 41 und dem Zusammenkommen der Stirnseiten 36 und 51 der Schiene 3 und des Schiebers 5 aufgeschoben. Mit dem so vorbereiteten Drainage-Set kann dann die Schiene 3 optimal plaziert werden, wobei die Schiene 3 mit dem Schieber 5 vorwärts und dem Führungsmandrin zurück bewegt wird, das Verdrehen der Schiene 3 über den Schieber 5 und das Lösen der Schiene 3 durch eine axiale Relativ-Bewegung des Führungsmandrins 4 gegen den Schieber 5 erfolgt, wobei der Schieber 5 auf Druck und der Führungsmandrin auf Zug belastet wird. Dabei bewirkt der Druck des Schiebers 5 auf die Ureter-Schiene 3 deren Aufweitung, so daß sich der Reibschluß zwischen der Schiene 3 und der Verdickung 41 verringert, so daß ein Lösen der Verdickung 41 von der Schiene 3 erleichtert und das Herausziehen des Führungsmandrins 4 aus der Schiene 3 ermöglicht wird.

Figur 9 zeigt das drehfeste Zusammenwirken der Verdickung 41 mit dem distalen Ende 51 des Schiebers 5 gemäß der zuletzt beschriebenen vorzugsweisen Ausführung, wobei diese Darstellung jedoch keine Einschränkung auf diesen Querschnitt darstellen soll.

Figur 10 zeigt deshalb als Beispiel einen denkbaren Querschnitt der Verdickung 41. In dieser Ausführung weist die Verdickung 41 eine zweizählige Rotationssymmetrie bezüglich ihrer Längsachse auf.

Die Ureter-Schiene kann Längen von 16 bis 32 cm aufweisen, wobei die Verdickung 41, wie gesagt, vom distalen Ende 42 des Führungsmandrins 4 jeweils einen Abstand aufweist, im wesentlichen der der Länge der entsprechenden Drainage-Schiene 3 (mit den oben genannten Besonderheiten hinsichtlich distal geschlossener bzw. offener Schiene und/oder drehfester Verbindung Schieber/Verdickung) entspricht. Die Länge der Verdickung 41 kann vorzugsweise 0,5 bis 2 cm aufweisen und ihre Dicke im Bereich 1 bis 2 mm liegen. Der Führungsmandrin selbst hat eine Stärke von weniger als 1 mm, seine Gesamtlänge kann in weiten Bereichen variiert werden, beispielsweise zwischen 0,5 und 1 m. Beim offenem distalen Ende 34 der Schiene 3 ragen vorzugsweise wenige mm bis 1 cm aus der Schiene 3 heraus. Die sonstigen Dimensionen sind übliche und an sich bekannt.

## Patentansprüche

1. Drainage-Set, insbesondere zur Ureter-Drainage, mit einer Drainage-Schiene (3), einem Führungsmandrin (4) und einem Schieber (5), wobei der Führungsmandrin (4) eine fest auf ihm ausgebildete Verdickung (41) aufweist, dadurch gekennzeichnet, daß auf die Verdickung (41) die Drainage-Schiene (3) mit ihrem Lumen kraftschlüssig aufschiebbar ist, und daß der Lumendurchmesser des Schiebers (5) größer oder gleich dem Durchmesser der Verdickung (41), aber geringer als der Außendurchmesser der Drainage-Schiene (3) ist.

2. Drainage-Set nach Anspruch 1, dadurch gekennzeichnet, daß die Verdickung (41) aus einer am Führungsmandrin (4) befestigten, diesen umgebenden Hülse besteht.

3. Drainage-Set nach Anspruch 1, dadurch gekennzeichnet, daß die Verdickung (41) einstückig mit dem Führungsmandrin (4) ausgebildet ist.

4. Drainage-Set nach Anspruch 1 oder 2, dadurch gekennzeichnet, daß die Verdickung (41) durch Kleben mit dem Führungsmandrin (4) verbunden ist.

5. Drainage-Set nach Anspruch 1 oder 2, dadurch gekennzeichnet, daß die Verdickung (41) durch Anschmelzen und anschließendem Erstarren mit dem Führungsmandrin (4) verbunden ist.

6. Drainage-Set nach einem der Ansprüche 1 bis 5, dadurch gekennzeichnet, daß sich die Verdickung (41) an ihren Stirnseiten konisch verjüngt.

7. Drainage-Set nach einem der vorangehenden Ansprüche, dadurch gekennzeichnet, daß die Verdickung (41) eine n-zählige Rotationssymmetrie parallel zur Längsachse des Führungsmandrins (4) aufweist, wobei n eine natürliche endliche Zahl ist.

8. Drainage-Set nach einem der vorangehenden Ansprüche, dadurch gekennzeichnet, daß die Verdickung (41) einen mehrkantigen Querschnitt aufweist.

9. Drainage-Set nach Anspruch 7 oder 8, dadurch gekennzeichnet, daß die Kontur des lichten Querschnitts des Schiebers (5) an seinem distalen Ende (51) der Kontur der Verdickung (41) angepaßt ist, so daß eine in das distale Ende (51) des Schiebers (5) eingesteckte Verdickung (41) zwar axial verschieblich aber drehfest mit diesem verbunden ist.

10. Drainage-Set nach einem der vorangehenden Ansprüche, dadurch gekennzeichnet, daß die Drainage-Schiene (3) im unbelasteten Zustand zumindestens ein, vorzugsweise zwei gekrümmte Ende(n) (32,33) aufweist.

11. Drainage-Set nach einem der vorangehenden Ansprüche, dadurch gekennzeichnet, daß die Drainage-Schiene (3) ein stirnseitig offenes distales Ende (34) aufweist.

12. Drainage-Set nach einem der vorangehenden Ansprüche, dadurch gekennzeichnet, daß der Führungsmandrin (4) hohl ausgebildet ist und daß sich durch ihn eine axial relativ verschiebbare Seele (44) erstreckt.

13. Drainage-Set nach Anspruch 12, dadurch gekennzeichnet, daß die Seele (44) proximal mit einem Handhabungsansatz (47) fest verbunden ist, der eine der Schiene (3) entsprechende Stärke aufweist.

14. Drainage-Set nach Anspruch 12 oder 13, dadurch gekennzeichnet, daß die Seitenelastizität des Führungsmandrins (4) in seinem distalen Endbereich (42) bei aus diesem Bereich herausgezogener Seele (44) geringer ist als die Rückstellkraft des hakenförmigen distalen Endes (34) der Drainage-Schiene (3).

## Claims

1. Drainage set, particularly for ureter drainage, with a drainage rail (3), a guide mandrel (4) and a slide (5), the guide mandrel (4) having a thickened portion fixed thereon, characterized in that the internal diameter of the drainage rail (3) can be frictionally slid onto the thickened portion (41) and that the internal diameter of the slide (5) is equal to or larger than the diameter of the thickened portion (41), but smaller than the external diameter of the drainage rail (3).

2. Drainage set according to claim 1, characterized in that the thickened portion (41) comprises a sleeve fixed to the guide mandrel (4) and surrounding the latter.

3. Drainage set according to claim 1, characterized in that the thickened portion (41) is constructed in one piece with the guide mandrel (4).

4. Drainage set according to claim 1 or 2, characterized in that the thickened portion (41) is connected by adhesion to the guide mandrel (4).

5. Drainage set according to claim 1 or 2, characterized in that the thickened portion (41) is connected to the guide mandrel (4) by melting and subsequent solidification.

6. Drainage set according to one of the claims 1 to 5, characterized in that the end faces of the thickened portion (41) are conically tapered.

7. Drainage set according to one of the preceding claims, characterized in that the thickened portion (41) has a n-fold rotational symmetry parallel to the longitudinal axis of the guide mandrel (4), n being a natural, finite number.

8. Drainage set according to one of the preceding claims, characterized in that the thickened portion (41) has a polygonal cross-section.

9. Drainage set according to claim 7 or 8, characterized in that the contour of the internal cross-section of the slide (5) is adapted at its distal end (51) to the contour of the thickened portion (41), so that although a thickened portion (41) inserted in the distal end (51) of the slide (5) is axially displaceable, it is connected in non-rotary manner thereto.

10. Drainage set according to one of the preceding claims, characterized in that, in the unloaded state, the drainage rail (3) has at least one and preferably two curved ends (32, 33).

11. Drainage set according to one of the preceding claims, characterized in that the drainage rail (3) has a frontally open distal end (34).

12. Drainage set according to one of the preceding claims, characterized in that the guide mandrel (4) is hollow and that an axially relatively displaceable core (44) extends through it.

13. Drainage set according to claim 12, characterized in that the core (44) is fixed at the proximal side to a handling attachment (47), which has a thickness corresponding to the rail (3).

14. Drainage set according to claim 12 or 13, characterized in that the lateral elasticity of the guide mandrel (4) in its distal end region (42) in the case of a core (44) being drawn out of this area is lower than the restoring force of the hook-like distal end (34) of the drainage rail (3).

## Revendications

1. Ensemble de drainage, en particulier drainage de l'urètre, comprenant un tube de drainage (3), un mandrin de guidage (4) et un coulisseau (5), le mandrin de guidage (4) présentant un renflement (41) conformé solidairement sur lui, caractérisé en ce que le tube de drainage (3) est coulissant sur le renflement (41) par son ouverture intérieure par l'effet d'une force élastique de retrecissement, et en ce que le diamètre de l'ouverture du coulisseau (5) est supérieur ou égal au diamètre du renflement (41), mais inférieur au diamètre extérieur du tube de drainage (3).

2. Ensemble de drainage selon la revendication 1, caractérisé en ce que le renflement (41) consiste en un manchon monté solidaire sur le mandrin de guidage (4) et entourant celui-ci.

3. Ensemble de drainage selon la revendication 1, caractérisé en ce que le renflement (41) est conformé d'une seule pièce avec le mandrin de guidage (4).

4. Ensemble de drainage selon l'une des revendications 1 ou 2, caractérisé en ce que le renflement (41) est solidarisé au mandrin de guidage (4) par collage.

5. Ensemble de drainage selon l'une des revendications 1 ou 2, caractérisé en ce que le renflement (41) est solidarisé au mandrin de guidage (4) par fusion puis solidification.

6. Ensemble de drainage selon l'une des revendications 1 à 5, caractérisé en ce que le renflement (41) se rétrécit de façon conique au niveau de ses parties frontales.

7. Ensemble de drainage selon l'une quelconque des revendications précédentes, caractérisé en ce que le renflement (41) présente une symétrie par rotation d'ordre *n* parallèlement à l'axe longitudinal du mandrin de guidage (4), *n* étant un nombre fini naturel.

8. Ensemble de drainage selon l'une quelconque des revendications précédentes, caractérisé en ce que le renflement présente une section polygonale.

9. Ensemble de drainage selon l'une des revendications 7 ou 8, caractérisé en ce que le contour de la section transversale du coulisseau (5) au niveau de son extrémité distale (51) est adapté au contour du renflement (41), de telle sorte qu'un renflement (41) introduit dans l'extrémité distale (51) du coulisseau (5) peut coulisser axialement, mais est immobilisé en rotation avec lui.

10. Ensemble de drainage selon l'une quelconque des revendications précédentes, caractérisé en ce que le tube de drainage (3) présente à l'état de repos au moins une, et de préférence deux, extrémités courbées (32,33).

11. Ensemble de drainage selon l'une quelconque des revendications précédentes, caractérisé en ce que le tube de drainage (3) présente une extrémité distale (34) ouverte frontalement.

12. Ensemble de drainage selon l'une quelconque des revendications précédentes, caractérisé en ce que le mandrin de guidage (4) est creux et en ce qu'une âme (44) coulissante s'étend axialement le long du mandrin.

13. Ensemble de drainage selon la revendication 12, caractérisé en ce que l'âme (44) est reliée au niveau de l'extrémité proximale à un élément de préhension (47) qui présente une épaisseur correspondant au tube (3).

14. Ensemble de drainage selon l'une des revendications 12 ou 13, caractérisé en ce que l'élasticité latérale du mandrin de guidage (4) au niveau de son extrémité distale (42), l'âme (44) étant retirée de cette partie, est inférieure à la force de rappel de l'extrémité distale (34) en forme de crochet du tube de drainage (3).
